# EUROPEAN PATENT APPLICATION

(11) **EP 3 069 660 A1**
(43) Date of publication of application: **21.09.2016**
(21) Application number: 15159213.6
(22) Date of filing: 16.03.2015
(51) Int. Cl.: A61B 6/04, A61N 5/10

(54) **Method for recovering a position of a patient**

(71) Applicant: Siemens Healthcare GmbH, 91052 Erlangen (DE)
(72) Inventor: Colombo, Vittorio, 90765 Fürth-Poppenreuth (DE); Mohr, Cecile, 91052 Erlangen (DE); Ripert, Alexandre, 91052 Erlangen (DE)

(57) **Abstract**

The present invention relates to a method for reproducibly recovering a position of a patient in a medical examination performed in an imaging device, comprising the steps of capturing (S101) an image dataset of a positioning device for positioning the patient in the medical examination; detecting (S102) a type and position of the positioning device on the basis of the image dataset; and storing (S103) the type and position of the positioning device in the image dataset of the patient.

## Description

The present invention relates to a method for reproducibly recovering a position of a patient in a medical examination performed in an imaging device and a medical imaging device for examining a patient.

Patient positioning is a fundamental step in radiation therapy planning. During planning at computer tomography CT, magnetic resonance MR or positron emission tomography-computer tomography PET-CT scanners, patients are positioned with the help of various positioning devices, such as breast boards or belly boards.

To ensure the most accurate radiotherapy treatment, there is a need to document the positioning accessories used during planning on the scanner, and to ensure that the documentation is forwarded over the Oncology Information System to the linear accelerator LINAC for reproducible positioning during treatment with radiation, like in external beam radiation therapy or particle therapy.

It is an object of the present invention to enable a precise positioning of a patient in a medical examination.

This object is solved by the subject-matter according to the independent claims. Further advantageous embodiments are given in the dependent claims, the description and the figures.

According to a first aspect this object is solved by a method for reproducibly recovering a position of a patient in a medical examination performed in an imaging device, comprising the steps of capturing an image dataset of a positioning device for positioning the patient in the medical examination; detecting a type and position of the positioning device on the basis of the image dataset; and storing the type and position of the positioning device in the image dataset of the patient. The method has the technical advantage that the position of a patient can be reproduced with high accuracy in different medical devices, like a linear accelerator. Manually typing the positioning device information into the Oncology Information System is not necessary. Therefore errors in entering the data can be avoided.

In an embodiment of the method the positioning device is a breast board for positioning a breast of the patient, a belly board for positioning a belly of the patient or a knee rest for positioning a knee of the patient. In this embodiment the technical advantage is achieved that different body part can be positioned exactly.

In a further embodiment of the method the type and position of the positioning device is detected on the basis of a comparison between the image dataset and a pre-stored dataset of the positioning device. In this embodiment the technical advantage is achieved that the accuracy of detecting a type and position of the positioning device is increased.

In a further embodiment of the method datasets of a plurality of positioning devices are pre-stored in a database. In this embodiment the technical advantage is achieved that different types of positioning devices can be easily identified.

In a further embodiment of the method the dataset of the positioning device is a three-dimensional dataset for rendering a three-dimensional shape of the positioning device. In this embodiment the technical advantage is achieved that the position can be determined under consideration of the shape of the body model.

In a further embodiment of the method the dataset of the positioning device is created by imaging the positioning device by the imaging device. In this embodiment the technical advantage is achieved that the datasets of various types of positioning devices can be determined precisely.

In a further embodiment of the method detecting the type and position of the positioning device is performed on the basis of fitting a digital model of the positioning device to the image dataset. In this embodiment the technical advantage is achieved that the type and position of the positioning device can be determined easily with high precision.

In a further embodiment of the method capturing the image dataset is based on computer tomography scanning, a magnetic resonance scanning or a positron emission tomography-computer tomography scanning. In this embodiment the technical advantage is achieved that a detailed image of the positioning device and the patient can be achieved.

In a further embodiment of the method the image dataset including the type and position of the positioning device is sent over a network to an external storage system. In this embodiment the technical advantage is achieved that the image dataset can be transferred to an oncology information system.

According to a second aspect this object is solved by a medical imaging device for examining a patient, comprising a capturing means for capturing an image dataset of a positioning device for positioning the patient in the medical examination; a detecting means for detecting a type and position of the positioning device on the basis of the image dataset; and a storing means for storing the type and position of the positioning device in the image dataset of the patient. The medical imaging device includes the same advantages as the method according to the first aspect.

In an embodiment of the medical imaging device the medical imaging device is a computer tomography scanner, a magnetic resonance scanner or a positron emission tomography-computer tomography scanner. In this embodiment also the technical advantage is achieved that a detailed image of the positioning device and the patient can be achieved.

In a further embodiment of the medical imaging device the detecting means is configured to detect the type and the position of the positioning device on the basis of a comparison between the image dataset and a pre-stored dataset of the positioning device. In this also embodiment the technical advantage is achieved that the accuracy of detecting a type and position of the positioning device is increased.

In a further embodiment of the medical imaging device the detecting means is configured to detect the type and the position of the positioning device on the basis of fitting a model of the positioning device to the image dataset. In this embodiment the technical advantage is achieved that the type and position of the positioning device can be determined easily with high precision.

In a further embodiment of the medical imaging device the medical imaging device comprises a database, in which datasets of a plurality of positioning devices are pre-stored. In this embodiment the technical advantage is achieved that different types of positioning devices can be easily identified.

In a further embodiment of the medical imaging device the medical imaging device comprises a sending means for sending the image dataset including the type and position of the positioning device over a network to an external storage system. In this embodiment the technical advantage is achieved that the image dataset can be transferred to an oncology information system.

Further features, details and advantages of the aforementioned subject-matter are shown in the figures and described below.
- Fig. 1: shows a block diagram of the method for reproducibly recovering a position; and
- Fig. 2: shows a schematic view of a medical imaging device for examining a patient.

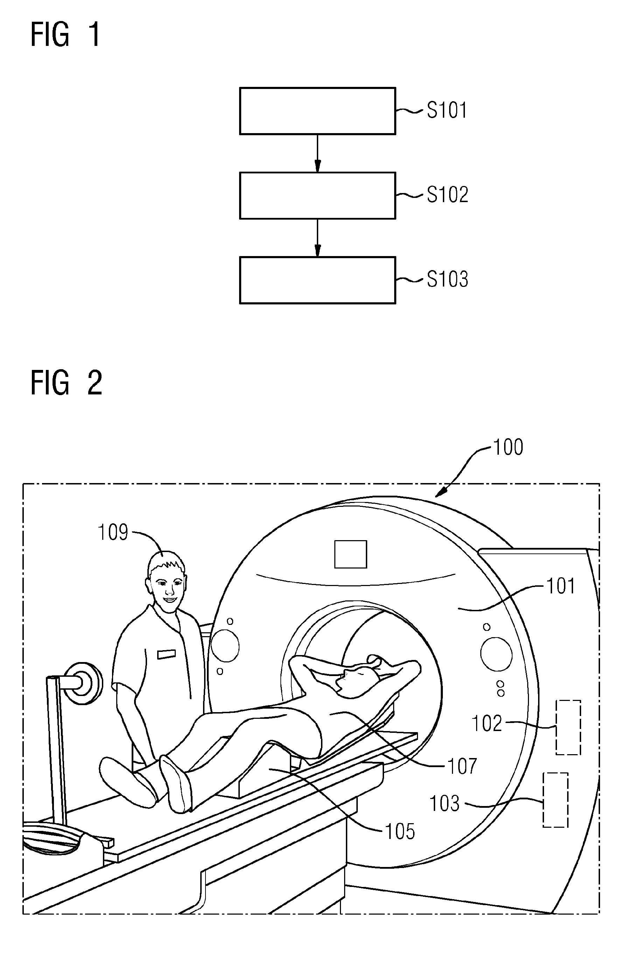

Fig. 1 shows a block diagram of the method for reproducibly recovering a position of a patient in a medical examination performed in an imaging device. In the medical examination the replaceable positioning device is used for positioning and fixing a patient with respect to the imaging device. The positioning device can for example be a breast board for positioning a breast of the patient, a belly board for positioning a belly of the patient or a knee rest for positioning a knee of the patient.

The method comprises the steps S101 of capturing an image dataset of a positioning device for positioning the patient in the medical examination; the step S102 of detecting a type and position of the positioning device on the basis of the image dataset; and the step S103 of storing the type and position of the positioning device in the image dataset of the patient.

Capturing an image dataset is performed by scanning a patient in combination with the positioning device used in the medical examination. Consequently the image dataset does not only comprise information about a patient but also information about the positioning device. Thus, detecting the type and position of the positioning device can be based on a comparison or analysis of pre-stored datasets of the positioning device and the image dataset. Detecting the type and position of the positioning device can also be based on fitting a digital model of the positioning device to the image dataset. In this fitting a model of the positioning device is stretched or moved in order to comply with the positioning device as captured in the image dataset.

A pre-stored dataset of the positioning device can be obtained during an initial acceptance testing of the medical device by the clinic staff, such as a physicist. In this acceptance testing different positioning devices are scanned by the imaging device without a patient. In addition a description can be assigned in the software to the scanned positioning device, like breast board, knee rests or lock bars. The description includes information about a position, such as three-dimensional coordinates or an angulation. In this way the datasets of a plurality of positioning devices are pre-stored in a database or storage that can be accessed by the imaging device.

Thus, types and positions of the positioning devices are stored during acceptance testing. These datasets are then stored within the imaging device software in a database in order to create a catalog comprising images of multiple positioning devices. This pre-stored catalog will be used with patients for automatic recognition of the positioning devices.

When a patient is examined by the imaging device, the topogram or scout images are acquired at the beginning of the scanning procedure in a special detection mode for the type and position of the positioning device. After the initial acceptance testing the software provides a software and acquisition mode solution for detection of the type and position of the positioning device with a patient in a clinical setting.

The software of the imaging device reconstructs the images. Using the images from the pre-stored catalog of the positioning devices the type of the positioning device and its position for the current patient setup is automatically detected.

The software suggests to the user a correct positioning device and documents it in the patient file for further usage. The software also reconstructs in three dimensions a volume rendered image which helps to document a patient position.

Then the software solution documents and exports automatically the type and position of the positioning device to further IT-systems of the clinic. The software documents the type and position, such as angulation, of the positioning device in a report, e.g. a pdf-file or a DICOM/DICOM RT-file. In addition it creates three-dimensional VRT images (VRT - Volume Rendering Technique) of the patient in a treatment position during the planning phase.

The software enables to store additional positioning pictures taken by a technologists or therapists and can associate further images, such as set-up pictures. The software sends the above mentioned information to the Oncology Information System either automatically or manually.

Fig. 2 shows a schematic view of a medical imaging device 100 for examining a patient 107 by a physician 109. The medical imaging device 100 is a computer tomography scanner that uses computer-processed X-rays to produce tomographic images in form of virtual slices of specific areas of the patient 107. This allows the physician 109 to see inside the object without cutting. The patient 107 is positioned within the computer tomography scanner using a replaceable positioning device 105, like a knee rest. Digital geometry processing is used to generate a three-dimensional image dataset of the inside of the patient 107 and the positioning device 105 from a large series of two-dimensional radiographic images taken around a single axis of rotation.

Another medical imaging device 100 is a magnetic resonance scanner using strong magnetic fields and radio waves to form images of the body of the patient 107. The magnetic resonance scanner forms a strong magnetic field around the area to be imaged.

In the medical application protons as contained in water or polymers are used to create a signal that is processed to form an image of the patient 107 and the positioning device 105. First, energy from an oscillating magnetic field is temporarily applied to the patient at the appropriate resonant frequency. The excited hydrogen atoms emit a radio frequency signal which is measured by a receiver coil. This signal is detected to create a three-dimensional image dataset of the inside of the patient 107 and the positioning device 105.

However, in general the imaging device 100 can also be a positron emission tomography-computer tomography scanner or a different imaging device.

In this way a capturing means 101 obtains the three dimensional image dataset of the positioning device 105 for positioning the patient 107 in the medical examination. Consequently, the image dataset comprises information about the positioning device 105 as well as the patient 107. The capturing means 101, like an imaging unit, comprises a detector for scanning the patient.

A detecting means 102 serves for detecting a type and position of the positioning device 105 on the basis of the image dataset. The detecting means 102 comprises a processor and a storage for processing the image dataset. The type and position of the positioning device 105 can be determined by performing a pattern or image analysis of the three dimensional image dataset. For example a pre-stored three dimensional digital model of the positioning device 105 is compared and fitted to the positioning device 105 as captured in the image dataset. By moving, rotating or stretching the digital model the position and orientation of the positioning device can be determined.

In addition the medical imaging device 100 comprises a storing means 103 for storing the type and position of the positioning device 105 in the image dataset of the patient 107. The detecting means 102 and storing means 103 can be realized by a computer, on which a suitable software is running. Finally the image dataset including the type and position of the positioning device 105 is sent over a network to an external storage system, like the Oncology Information System. This image dataset can again be used for re-positioning the patient in a further examination using the same type and position of the positioning device 105, like for example linear accelerator LINAC.

The method and the medical imaging device enables an automatic and image based detection of the positioning devices and their positions with respect to the patient. Therefore, the method leads to several technical advantages. It allows the clinical staff to focus on the patient during the therapy planning phase. Since the method being automated, it considerably reduces the possibility of human errors. Further it brings a potentially higher level of accuracy and reproducibility of the patient positioning, during planning phase at the scanner and during treatment and the linear accelerator.

The scope of the invention is defined by the claims and is not restricted by special features discussed in the description or shown in the figures. All features discussed with respect to different embodiments can be combined variously and independently in order to simultaneously realize their technical effect.

## Claims

1. Method for reproducibly recovering a position of a patient (107) in a medical examination performed in an imaging device (100), comprising the steps:
capturing (S101) an image dataset of a positioning device (105) for positioning the patient (107) in the medical examination;
detecting (S102) a type and position of the positioning device (105) on the basis of the image dataset; and
storing (S103) the type and position of the positioning device (105) in the image dataset of the patient (107).

2. Method according to claim 1, wherein the positioning device (105) is a breast board for positioning a breast of the patient, a belly board for positioning a belly of the patient or a knee rest for positioning a knee of the patient.

3. Method according to one of the preceding claims, wherein the type and position of the positioning device (105) is detected on the basis of a comparison between the image dataset and a pre-stored dataset of the positioning device (105).

4. Method according to claim 3, wherein datasets of a plurality of positioning devices (105) are pre-stored in a database.

5. Method according to claim 3 or 4, wherein the dataset of the positioning device (105) is a three-dimensional dataset for rendering a three-dimensional shape of the positioning device (105).

6. Method according to one of the claims claim 3 to 5, wherein the dataset of the positioning device (105) is created by imaging the positioning device (105) by the imaging device (100).

7. Method according to one of the preceding claims, wherein detecting the type and position of the positioning device (105) is performed on the basis of fitting a digital model of the positioning device (105) to the image dataset.

8. Method according to one of the preceding claims, wherein capturing the image dataset is based on computer tomography scanning, a magnetic resonance scanning or a positron emission tomography-computer tomography scanning.

9. Method according to one of the preceding claims, wherein the image dataset including the type and position of the positioning device (105) is sent over a network to an external storage system.

10. Medical imaging device (100) for examining a patient, comprising:
a capturing means (101) for capturing an image dataset of a positioning device (105) for positioning the patient (107) in the medical examination;
a detecting means (102) for detecting a type and position of the positioning device (105) on the basis of the image dataset; and
a storing means (103) for storing (S103) the type and position of the positioning device (105) in the image dataset of the patient (107).

11. Medical imaging device (100) according to claim 10, wherein the medical imaging device (100) is a computer tomography scanner, a magnetic resonance scanner or a positron emission tomography-computer tomography scanner.

12. Medical imaging device (100) according to claim 10 or 11, wherein the detecting means (102) is configured to detect the type and the position of the positioning device (105) on the basis of a comparison between the image dataset and a pre-stored dataset of the positioning device (105).

13. Medical imaging device (100) according to one of the claims 10 to 12, wherein the detecting means (102) is configured to detect the type and the position of the positioning device (105) on the basis of fitting a model of the positioning device (105) to the image dataset.

14. Medical imaging device (100) according to one of the claims 10 to 13, wherein the medical imaging device (100) comprises a database, in which datasets of a plurality of positioning devices (105) are pre-stored.

15. Medical imaging device (100) according to one of the claims 10 to 14, wherein the medical imaging device (100) comprises a sending means for sending the image dataset including the type and position of the positioning device (105) over a network to an external storage system.
